# EUROPÄISCHE PATENTSCHRIFT

(11) **EP 3 838 130 B1**
(45) Veröffentlichungstag und Bekanntmachung des Hinweises auf die Patenterteilung: **28.06.2023**
(21) Anmeldenummer: 20214311.1
(22) Anmeldetag: 15.12.2020
(51) Int. Cl.: A61B 5/026, A61B 5/0295, A61B 5/00, A61B 5/02, A61F 13/08

(54) **SYSTEM FÜR EINE KOMPRESSIONSTHERAPIE UMFASSEND EINEN PHOTOPLETHYSMOGRAPHISCHEN SENSOR**
SYSTEM FOR COMPRESSION THERAPY COMPRISING A PHOTOPLETHYSMOGRAPHIC SENSOR
SYSTÈME POUR UNE THÉRAPIE DE COMPRESSION COMPRENANT UN CAPTEUR PHOTOPLÉTHYSMOGRAPHIQUE

(30) Priorität: 19.12.2019 DE 102019135071
(43) Veröffentlichungstag der Anmeldung: 23.06.2021
(73) Patentinhaber: Paul Hartmann AG, 89522 Heidenheim (DE)
(72) Erfinder: SMOLA, Hans, 66121 Saarbrücken (DE)

(56) Entgegenhaltungen:
- DE-A1-102007 034 874
- US-A1- 2015 297 132
- CORNWALL J V ET AL: "GRADUATED COMPRESSION AND ITS RELATION TO VENOUS REFILLING TIME", BRITISH MEDICAL JOURNAL, B M J GROUP, GB, Bd. 295, Nr. 6606, 31. Oktober 1987 (1987-10-31), Seiten 1087-1090, XP008062817, ISSN: 0267-0623
- STEIN PAUL D ET AL: "Effect of Graduated Compression Stockings on Venous Blood Velocity in Supine Resting Hospitalized Patients", CLINICAL AND APPLIED THROMBOSIS/HEMOSTASIS, Bd. 20, Nr. 7, 27. Februar 2013 (2013-02-27), Seiten 693-697, XP055798434, US ISSN: 1076-0296, DOI: 10.1177/1076029613479821

## Beschreibung

Die Erfindung betrifft ein System für eine Kompressionstherapie und ein dafür vorgesehenes Computerprogrammprodukt.

Die Photoplethysmographie ist eine Untersuchungsmethode in der Diagnostik der Veneninsuffizienz. Sie wird beispielsweise in den Artikeln "Graduated compression and its relation to venous refilling time" (Cornwall et al., British Medical Journal, Band 295, Oktober 1987) und "Effect of graduated compression stockings on venous blood velocity in supine resting hospitalized patients" (Stein et al., Clinical and Applied Thrombosis/Hemostasis, Band 20(7), 2014) sowie der DE102007034874A1 beschrieben. Bei der Photoplethysmographie führt der Patient Bewegungen (zum Beispiel Zehenstände oder ähnliches) aus, um die Venen im Unterschenkel durch die Aktivität der Muskulatur zu entleeren. Dann ruht der Patient wieder, so dass sich die Venen erneut mit Blut füllen können. Die Zeitdauer bis zum Erreichen des ursprünglichen Füllungszustandes der Venen mit Blut, die sogenannte Wiederauffüllungszeit, wird als diagnostisches Mittel eingesetzt. So beträgt die Wiederauffüllungszeit bei (venen)gesunden Probanden 25 Sekunden oder mehr. Ein niedrigerer Wert für die Wiederauffüllungszeit wird als ein Hinweis auf eine Veneninsuffizienz angesehen. Technisch gesehen kommt bei der Photoplethysmographie zur Bestimmung der Wiederauffüllungszeit ein Infrarot-Sensor (IR-Sensor) zum Einsatz. Der Infrarot-Sensor bestrahlt eine Stelle am Unterschenkel des Patienten mit infrarotem Licht, welches dann in Abhängigkeit von der Blutmenge in den Venen unterschiedlich stark wieder zum Sensor reflektiert wird. Der Sensor erfasst das reflektierte infrarote Licht und anhand der erhaltenen Messkurve kann die Wiederauffüllungszeit ermittelt werden.

Zur Behandlung der Veneninsuffizienz werden häufig Kompressionsbinden eingesetzt. Aus dem Stand der Technik ist es bereits bekannt, Kompressionsbinden mit Drucksensoren auszustatten. Dies ist zum Beispiel in der US2015/0297132A1 beschrieben. Die ermittelten Druckwerte sollen Auskunft darüber geben, ob die Kompressionsbinde korrekt am Körper des Patienten angelegt ist, so dass diese therapeutisch wirksam sein kann. Zweifellos kann mit derartigen Drucksensoren ein Fortschritt in der Kompressionstherapie erreicht werden. Allerdings kann die Wirksamkeit der Kompressionstherapie mit Druckmessungen lediglich indirekt überprüft werden. Zudem können die erforderlichen Druckwerte für eine therapeutische Wirksamkeit der Kompressionstherapie von Patient zu Patient unterschiedlich sein. Wünschenswert wäre demnach eine Möglichkeit, mit der die Wirksamkeit und Sicherheit einer Kompressionstherapie direkter und in weitestgehend gleicher Art und Weise für möglichst viele Patienten überprüft werden kann. Eine solche Möglichkeit anzugeben stellte die Aufgabe der vorliegenden Erfindung dar. Die Aufgabe wird mit einem System nach Anspruch 1 und einem Computerprogrammprodukt nach Anspruch 15 gelöst.

So umfasst das System für eine Kompressionstherapie ein Kompressionsmittel, einen photoplethysmographischen Sensor (PPG Sensor) und einen Computer. Messwerte des photoplethysmographischen Sensors können an den Computer übertragen werden. Weiterhin umfasst der Computer Mittel zur Ausführung eines Verfahrens zur Überprüfung der Kompressionstherapie, wobei das Verfahren die folgenden Schritte umfasst:
i. Empfangen von Messwerten des photoplethysmographischen Sensors während der Kompressionstherapie,
ii. Bestimmen einer Wiederauffüllungszeit anhand der empfangenen Messwerte des photoplethysmographischen Sensors,
iii. Prüfen, ob die bestimmte Wiederauffüllungszeit eine vorgegebene Untergrenze unterschreitet,
iv. optional Ausgabe eines Alarms, wenn die Prüfung ergibt, dass die vorgegebene Untergrenze unterschritten wird.

Bei den zuvor genannten Mitteln des Computers handelt es sich insbesondere um einen Prozessor und ein Computerprogramm. Letzteres ist Gegenstand des Anspruchs 15 und wird im Folgenden noch genauer beschrieben. Photoplethysmographie kann nicht nur zu diagnostischen Zwecken zur Erkennung eines Venenleidens, sondern auch zur Überprüfung einer Kompressionstherapie verwendet werden. Denn die bei der

Photoplethysmographie gemessene Wiederauffüllungszeit sollte bei einem venenkranken Patienten durch die Kompressionstherapie jedenfalls dann verlängert werden, wenn die Kompressionstherapie therapeutisch wirksam ist und zu einer verbesserten Venenfunktion führt. Eine wirksame Kompressionstherapie sollte also zu einem normalen oder zumindest verbesserten Befund bei der Photoplethysmographie führen, was nach dem erfindungsgemäßen Gedanken als Kontrollmöglichkeit für die Effektivität der Kompressionstherapie herangezogen wird. Die Vorgehensweise bei der Überprüfung der Kompressionstherapie sieht dabei grundsätzlich folgendermaßen aus: Der Patient führt mit dem angelegten Kompressionstherapiesystem ein für die Photoplethysmographie übliches Bewegungsprogramm einschließlich einer Ruhephase durch. Das Kompressionstherapiesystem bestimmt auf Basis der erhobenen Messwerte die Wiederauffüllungszeit und prüft diese dahingehend, ob sie eine vorgegebene Untergrenze unterschreitet. Wenn dies der Fall ist, kann ein Alarm ausgegeben werden. Der Alarm weist den Patienten auf eine unzureichende Wirksamkeit der Kompressionstherapie hin. Die unzureichende Wirksamkeit könnte die Folge einer zu geringen Kompressionsleistung des Kompressionstherapiesystems sein. Eine solche zu geringe Kompressionsleistung kann beispielsweise auftreten, wenn eine als Kompressionsmittel verwendete Binde zu locker an dem zu behandelnden Körperteil angelegt worden ist. Indem die Binde dann nach der Alarmmeldung neu und unter höherem Zug angelegt wird, kann die Wirksamkeit der Kompressionstherapie verbessert beziehungsweise wiederhergestellt werden. Insgesamt kann die Wirksamkeit der Kompressionstherapie damit in vielen Fällen sehr direkt und einfach überprüft werden.

Das erfindungsgemäße Kompressionstherapiesystem könnte auch noch mit einem Drucksensor ausgestattet werden. Zwingend erforderlich und vorgesehen ist dies jedoch nicht. Das System kann ebenso keinen Drucksensor umfassen.

Das Kompressionsmittel im Sinne der vorliegenden Erfindung ist insbesondere dafür vorgesehen an einem Unterschenkel eines Menschen angebracht zu werden. Das Kompressionsmittel kann eine Kompressionsbinde, ein Kompressionsschlauch oder ein Kompressionsstrumpf sein. Die Kompressionsbinde hat den Vorteil, dass sie sehr anpassungsfähig ist. Der Kompressionsschlauch und der Kompressionsstrumpf können leicht und schnell angelegt werden. Kompressionsbinden sind im Handel erhältlich, zum Beispiel von der Patentanmelderin Paul Hartmann AG (Heidenheim, Deutschland) unter dem Markennamen Pütter^{®}. Es können auch mehrere der zuvor genannten Kompressionsmittel miteinander kombiniert werden. Zum Beispiel kann eine Kompressionsbinde mit einem Kompressionsstrumpf kombiniert eingesetzt werden.

Üblicherweise umfasst der photoplethysmographische Sensor eine Lichtquelle für infrarotes Licht und einen Photodetektor. Die Lichtquelle ist vorzugsweise eine Leuchtdiode (lightemitting diode, LED). Der Photodetektor ist vorzugsweise eine Photodiode. Der Photodetektor kann das von dem Gewebe reflektierte infrarote Licht erfassen. Entsprechend können die anspruchsgemäßen Messwerte des photoplethysmographischen Sensors Messwerte des Photodetektors im Hinblick auf das reflektierte infrarote Licht umfassen.

Für die Handhabbarkeit ist es vorteilhaft, wenn das System eine Sensoreinheit mit einem Substrat umfasst, wobei der photoplethysmographische Sensor auf dem Substrat angeordnet ist. Bei dem Substrat der Sensoreinheit kann es sich um eine Leiterplatte, insbesondere um eine flexible Leiterplatte, handeln. Die Befestigung der Sensoreinheit am Körper des Patienten beziehungsweise an dem Kompressionsmittel kann erleichtert werden, indem die Leiterplatte flexibel ausgebildet ist. Die flexible Leiterplatte verbessert insbesondere auch den Tragekomfort des Kompressionstherapiesystems. Bevorzugt handelt es sich bei der flexiblen Leiterplatte um einen Polymerfilm, auf dem elektronische Komponenten aufgebracht, insbesondere aufgedruckt, sind.

Darüber hinaus kann die Sensoreinheit eine Datenerfassungseinheit umfassen. Die Datenerfassungseinheit ist dafür ausgebildet die Messwerte von dem photoplethysmographischen Sensor zu erfassen und an den Computer zu übertragen. Ebenso wie der Sensor kann die Datenerfassungseinheit auf dem Substrat der Sensoreinheit angeordnet sein. Sie kann aber auch mit einem Konnektor, beispielsweise einem Kabel, mit dem Substrat der Sensoreinheit verbunden sein. Die Datenerfassungseinheit kann einen Microcontroller, einen Vorverstärker und einen Analog-Digital-Wandler umfassen. Weitere mögliche elektronische Komponenten der Datenerfassungseinheit werden nachfolgend genannt. Somit kann die Datenerfassungseinheit für sich gesehen gleichfalls einen Computer darstellen. Es ist dann sogar möglich, dass die Datenerfassungseinheit den Computer bei der Ausführung einzelner Schritte des Überprüfungsverfahrens unterstützt.

Gemäß einer besonders bevorzugten Ausführungsform der Erfindung ist die Sensoreinheit adhäsiv ausgebildet und wird losgelöst von dem Kompressionsmittel bereitgestellt. Dafür umfasst die Sensoreinheit vorzugsweise eine adhäsive Rückschicht, wobei die Sensoreinheit dann in der Art eines Pflasters ausgestaltet sein kann. Diese Ausführungsform hat die besonderen Vorteile, dass sich die Sensoreinheit leicht und ohne zusätzliche Hilfsmittel (wie etwa einem Heftpflaster) am Körper des Patienten anbringen lässt und der Patient oder das Pflegepersonal frei wählen kann, an welcher Stelle die Sensoreinheit zur Erhebung der Messwerte platziert werden soll. Andererseits kann es auch vorgesehen sein, dass die Sensoreinheit mit dem Kompressionsmittel herstellerseitig verbunden ist. Dadurch kann das Anlegen des Systems vereinfacht werden und schneller erfolgen.

Vorzugsweise handelt es sich bei dem Computer um ein mobiles Gerät. Als mobiles Gerät kommt für die Erfindung insbesondere ein Smartphone oder ein Tablet in Betracht. Dadurch kann die Anzahl der elektronischen Komponenten minimiert werden, weil auf ein bereits vorhandenes, leistungsstarkes Gerät des Patienten oder des Pflegepersonals für die Datenauswertung und Datenanzeige zurückgegriffen werden kann. Zudem kann damit die Handhabung und der Tragekomfort des Kompressionstherapiesystems verbessert werden.

In einer einfachen Ausgestaltung der Erfindung werden die Messwerte des photoplethysmographischen Sensors an den Computer kabelgebunden übertragen. Viel vorteilhafter ist es aber, wenn die Übertragung der Messwerte des photoplethysmographischen Sensors an den Computer kabellos erfolgt, wobei die Übertragung vorzugsweise über eine Nahfeldkommunikation (near field communication, NFC), Bluetooth oder ein drahtloses lokales Netzwerk (wireless local area network, WLAN) stattfindet. Dadurch können die Messwerte an einen beliebigen Ort übertragen und die Handhabung sowie der Tragekomfort des Systems verbessert werden. Für den kabellosen Datenaustausch kann das Kompressionstherapiesystem einen oder mehrere NFC-Sendeempfänger, Bluetooth-Sendeempfänger und/oder WLAN-Sendeempfänger umfassen. Ein NFC-Sendeempfänger, ein Bluetooth-Sendeempfänger und/oder ein WLAN-Sendeempfänger kann insbesondere in der Datenerfassungseinheit und dem Computer enthalten sein.

Um die Datensicherheit des Kompressionstherapiesystems zu verbessern, wird außerdem vorgeschlagen, dass die Übertragung der Messwerte des photoplethysmographischen Sensors an den Computer verschlüsselt erfolgt. Die Verschlüsselung kann zum Beispiel von der Datenerfassungseinheit durchgeführt werden. Die Entschlüsselung der Messwerte kann von dem Computer durchgeführt werden.

Normalerweise verfügt der photoplethysmographische Sensor über eine eigene Energiequelle, wobei die Energiequelle vorzugsweise eine Batterie ist. Das heißt, dass das Kompressionstherapiesystem normalerweise weiterhin eine Energiequelle, vorzugsweise eine Batterie, für den photoplethysmographischen Sensor umfasst. Die Energiequelle kann von der Sensoreinheit umfasst und zum Beispiel auf deren Substrat angeordnet sein.

Zudem können vorteilhafte Ausführungsformen der Erfindung im Hinblick auf die Arbeitsweise des Computers angegeben werden. So wird zum Beispiel bevorzugt, dass das Empfangen der Messwerte des photoplethysmographischen Sensors unmittelbar nachdem die Messwerte von dem Sensor erfasst wurden erfolgt. Dadurch kann der Benutzer des Systems schnell über die Wirksamkeit der Kompressionstherapie informiert werden. Ebenso wird bevorzugt, dass das Empfangen und damit insbesondere auch das Erzeugen der Messwerte des photoplethysmographischen Sensors auf Befehl eines Benutzers hin erfolgt. Ein dauerhaftes Monitoring der Sensormesswerte ist vorliegend nämlich weder notwendig noch sinnvoll, da der Patient für das Überprüfungsverfahren ein spezielles Bewegungsprogramm mit einer sich daran anschließenden Ruhephase absolvieren muss, was er nur im Bedarfsfall und höchstens in regelmäßigen Abständen zur Kontrolle der Kompressionstherapie machen wird. Diese Ausgestaltung reduziert gleichzeitig den Energieverbrauch des photoplethysmographischen Sensors.

Die Untergrenze für die Wiederauffüllungszeit kann aus einem Bereich von 10 Sekunden bis 25 Sekunden, bevorzugt von 15 Sekunden bis 25 Sekunden, mehr bevorzugt von 20 Sekunden bis 25 Sekunden und besonders bevorzugt von 23 Sekunden bis 25 Sekunden ausgewählt sein. Insbesondere beträgt die Untergrenze für die Wiederauffüllungszeit 25 Sekunden, was einem Normalbefund entsprechen würde.

Erfindungsgemäß wird in dem Verfahren zur Überprüfung der Kompressionstherapie weiterhin geprüft, ob die bestimmte Wiederauffüllungszeit eine vorgegebene Obergrenze überschreitet. Dabei wird optional ein Alarm ausgegeben, wenn die Prüfung ergibt, dass die vorgegebene Obergrenze überschritten wird. Dadurch könnte auch eine durch eine zu hohe Kompression verursachte Mangeldurchblutung des behandelten Körperteils erkannt und die Sicherheit des Kompressionstherapiesystems verbessert werden. Das mit dem erfindungsgemäßen System durchzuführende Verfahren umfasst also die folgenden Schritte:
i. Empfangen von Messwerten des photoplethysmographischen Sensors während der Kompressionstherapie,
ii. Bestimmen einer Wiederauffüllungszeit anhand der empfangenen Messwerte des photoplethysmographischen Sensors,
iii. Prüfen, ob die bestimmte Wiederauffüllungszeit eine vorgegebene Untergrenze unterschreitet,
iv. optional Ausgabe eines Alarms, wenn die Prüfung ergibt, dass die vorgegebene Untergrenze unterschritten wird,
v. Prüfen, ob die bestimmte Wiederauffüllungszeit eine vorgegebene Obergrenze überschreitet,
vi. optional Ausgabe eines Alarms, wenn die Prüfung ergibt, dass die vorgegebene Obergrenze überschritten wird.

Die Schritte iii. und iv. können auch mit den Schritten v. und vi. in ihrer Reihenfolge vertauscht werden.

Die Obergrenze besitzt einen höheren Wert als die Untergrenze. Folglich könnte auch die Prüfung im Hinblick auf die Obergrenze entfallen, wenn die Prüfung im Hinblick auf die Untergrenze zuvor ergeben hat, dass die Untergrenze unterschritten wird. Weiterhin könnte auch die Prüfung im Hinblick auf die Untergrenze entfallen, wenn die Prüfung im Hinblick auf die Obergrenze zuvor ergeben hat, dass die Obergrenze überschritten wird.

Gemäß der Erfindung bilden die Untergrenze und die Obergrenze einen Bereich. Wenn die bestimmte Wiederauffüllungszeit in diesem Bereich liegt, wird die Kompressionstherapie als wirksam und sicher angesehen und es erfolgt keine Alarmierung. Liegt die bestimmte Wiederauffüllungszeit hingegen außerhalb des Bereiches, wird die Kompressionstherapie als unwirksam (insbesondere bei einer Unterschreitung der Untergrenze) und/oder unsicher (insbesondere bei einer Überschreitung der Obergrenze) angesehen und ein Alarm ausgegeben.

Die Obergrenze für die Wiederauffüllungszeit kann aus einem Bereich von 30 Sekunden bis 180 Sekunden, bevorzugt von 35 Sekunden bis 90 Sekunden, mehr bevorzugt von 40 Sekunden bis 90 Sekunden und besonders bevorzugt von 45 Sekunden bis 90 Sekunden ausgewählt sein. Insbesondere beträgt die Obergrenze für die Wiederauffüllungszeit 60 Sekunden. Bei einer Untergrenze von beispielsweise 25 Sekunden würde sich dann ein voreingestellter Bereich für die Wiederauffüllungszeit von 25 Sekunden bis 60 Sekunden ergeben.

Wenn die Untergrenze für die Wiederauffüllungszeit unterschritten oder die Obergrenze für die Wiederauffüllungszeit überschritten wird, können wie bereits dargestellt die optionalen Alarme ausgegeben werden. Dabei werden die Alarme insbesondere visuell und/oder akustisch ausgegeben.

Üblicherweise umfasst das System weiterhin ein Eingabemittel und ein Ausgabemittel. Das Eingabemittel und das Ausgabemittel sind vorzugsweise Bestandteile des Computers. Das Eingabemittel kann dazu dienen, Befehle oder Vorgaben des Benutzers entgegenzunehmen.

Das Ausgabemittel kann dazu dienen, die Messwerte anzuzeigen und den Alarm auszugeben. Als sowohl Eingabemittel als auch Ausgabemittel kann ein Berührungsbildschirm (touch screen) von beispielsweise einem Smartphone oder Tablet fungieren. Das Ausgabemittel kann auch ein Lautsprecher sein (akustischer Alarm). Jedes Smartphone oder Tablet verfügt heutzutage über einen Berührungsbildschirm und einen Lautsprecher.

Normalerweise umfasst das System auch einen Datenträger. Auch der Datenträger ist vorzugsweise Bestandteil des Computers. Der Datenträger kann dazu dienen, Computerprogramme und die empfangenen Messwerte zu speichern. Dadurch kann der Benutzer alle im Verlauf der Kompressionstherapie empfangenen Messwerte nachvollziehen. Bei dem Datenträger kann es sich um eine Festplatte, ein Halbleiterlaufwerk (solid-state-drive, SSD) oder eine SD-Karte (secure digital memory card) handeln. Das Kompressionstherapiesystem kann auch mehr als einen Datenträger besitzen. Insbesondere die zuvor genannte Datenerfassungseinheit kann auch einen Datenträger umfassen. Denkbar ist zudem, dass die Sensormesswerte von dem Kompressionstherapiesystem in einem onlinebasierten Datenspeicherdienst (Cloud) oder auf einem lokalen Netzlaufwerk gespeichert werden.

Offenbart wird ebenso ein computerimplementiertes, nicht unter die Erfindung fallendes, Verfahren zur Überprüfung einer
Kompressionstherapie. Dieses Verfahren umfasst die folgenden Schritte:
i. Erzeugen von Messwerten mit einem photoplethysmographischen Sensor während der Kompressionstherapie, um die Wirksamkeit der Kompressionstherapie zu überprüfen,
ii. Übertragen der Messwerte des photoplethysmographischen Sensors während der Kompressionstherapie an einen Computer,
iii. Bestimmen einer Wiederauffüllungszeit anhand der Messwerte des photoplethysmographischen Sensors durch den Computer,
iv. Prüfen durch den Computer, ob die bestimmte Wiederauffüllungszeit eine vorgegebene Untergrenze unterschreitet,
v. optional Ausgabe eines Alarms durch den Computer, wenn die Prüfung ergibt, dass die vorgegebene Untergrenze unterschritten wird,
vi. Prüfen durch den Computer, ob die bestimmte Wiederauffüllungszeit eine vorgegebene Obergrenze überschreitet,
vii. optional Ausgabe eines Alarms durch den Computer, wenn die Prüfung ergibt, dass die vorgegebene Obergrenze überschritten wird.

Im Rahmen des obigen Schrittes ii. werden die Messwerte des photoplethysmographischen Sensors von dem Computer empfangen.

Das computerimplementierte Verfahren kann vorteilhaft mit sämtlichen der vorliegend beschriebenen Ausführungsformen des Kompressionstherapiesystems durchgeführt werden.

Zudem betrifft die Erfindung ein Computerprogramm zur Durchführung des zuvor beschriebenen computerimplementierten Verfahrens. Beansprucht wird dabei ein Computerprogrammprodukt, umfassend Befehle, die bei der Ausführung des Programmes durch ein erfindungsgemäßes Kompressionstherapiesystem dieses veranlassen, die Schritte i. bis vii. (also alle Schritte), die Schritte iii. bis vii. oder zumindest die Schritte iii., iv und vi. des oben genannten computerimplementierten Verfahrens auszuführen. Beansprucht wird außerdem ein computerlesbarer Datenträger, auf dem ein solches Computerprogrammprodukt gespeichert ist. Der computerlesbare Datenträger kann hierbei insbesondere eine CD, DVD oder ein USB-Stick sein. Schließlich wird noch ein Datenträgersignal beansprucht, das ein solches Computerprogrammprodukt überträgt. Das Datenträgersignal kann zum Beispiel von einer onlinebasierten Vertriebsplattform (App Store wie beispielsweise Google Play) ausgehen und das Computerprogramm auf ein Smartphone oder ein Tablet zur Installation übertragen.

### Beispiele und Figuren

Mit den nachfolgend beschriebenen Beispielen und Figuren soll die Erfindung exemplarisch näher erläutert und veranschaulicht werden.

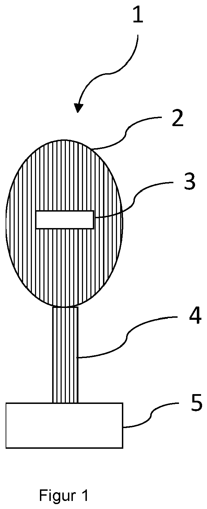

**Figur 1** zeigt eine Sensoreinheit 1. Die Sensoreinheit 1 ist ein Bestandteil einer ersten bevorzugten Ausführungsform des erfindungsgemäßen Kompressionstherapiesystems. Die Sensoreinheit **1** umfasst ein Substrat **2,** bei dem es sich um eine bevorzugt flexible Leiterplatte handelt. Auf dem Substrat **2** ist ein photoplethysmographischer Sensor **3** angeordnet. Üblicherweise besitzt der Sensor **3** eine Lichtquelle für infrarotes Licht sowie einen Photodetektor. Ein Konnektor **4** verbindet dann das Substrat **2** mit einer Datenerfassungseinheit **5.** Diese hat die Aufgabe, die Messwerte von dem Sensor **3** zu erfassen und an einen Computer (nicht dargestellt) kabellos, beispielsweise mittels Bluetooth oder NFC, zu übertragen. Hierfür kann die Datenerfassungseinheit **5** einen Datenträger und einen Bluetooth- oder NFC-Sendeempfänger umfassen (nicht dargestellt). Als Computer ist insbesondere ein Smartphone vorgesehen. Der Computer kann die empfangenen Messdaten dann in der erfindungsgemäß vorgesehen Art und Weise prüfen und den Anwender alarmieren, wenn eine unzureichende Wirksamkeit der Kompressionstherapie festgestellt wird.

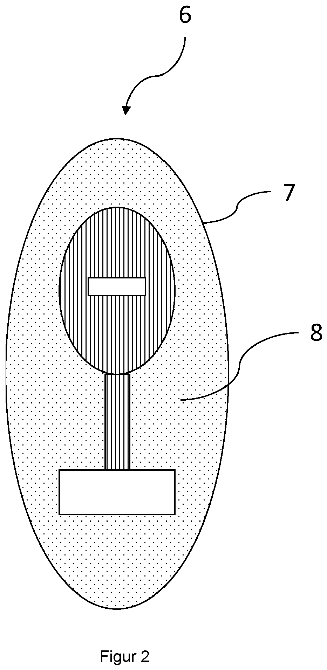

**Figur 2** zeigt eine Sensoreinheit **6** als Bestandteil einer zweiten bevorzugten Ausführungsform des erfindungsgemäßen Kompressionstherapiesystems. Die Sensoreinheit **6** unterscheidet sich von der Sensoreinheit 1 lediglich dadurch, dass sie in der Art eines Pflasters ausgebildet ist. Das pflasterartige Aussehen ergibt sich durch eine Rückschicht **7** ("backing"), welche auf einer Seite adhäsiv beziehungsweise klebend ausgerüstet ist und an dem Substrat **2,** dem Konnektor **4** und der Datenerfassungseinheit **5** zusätzlich befestigt ist. Mit dem freien, adhäsiven Randbereich **8** der Rückschicht **7** kann die Sensoreinheit **6** am Körper des Patienten angeklebt werden. Die Rückschicht **7** aus **Figur 2** hat eine ovale Form. Die Rückschicht **7** kann aber auch eine andere Form, beispielsweise eine rechteckige Form, haben.

### Anwendungsbeispiel

- Zu behandelndes Körperteil: Unterschenkel eines Menschen
- Bestandteile des eingesetzten Kompressionstherapiesystems:
   - Sensoreinheit 1 oder Sensoreinheit 2 aus Figur 1 beziehungsweise Figur 2
   - eine Kompressionsbinde (als Kompressionsmittel, welches losgelöst von der Sensoreinheit bereitgestellt wird)
   - ein Smartphone (als Computer mit einem Datenträger, einem Bluetooth- und NFC-Sendeempfänger, einem Berührungsbildschirm sowie einem Lautsprecher)
- Zeitpunkt der Kontrollmessungen: erfolgt auf Befehl eines Benutzers hin
- Ablauf der Behandlung (in chronologischer Reihenfolge):
   i. **Befestigen der Sensoreinheit** an einer Stelle des Unterschenkels, an der das venöse Blutvolumen gemessen werden soll. Für die Befestigung der Sensoreinheit 1 aus Figur 1 kann ein Heftpflaster aus dem medizinischen Bereich, zum Beispiel Omniplast^{®} von der Patentanmelderin, zu Hilfe genommen werden.
   ii. **Umwickeln des Unterschenkels mit der Kompressionsbinde.** Die Sensoreinheit ist dann zwischen Haut und Binde angeordnet. Der Zeitpunkt nach Anlegen der Kompressionsbinde kann als Beginn der Kompressionstherapie angesehen werden.
   iii. **Aktivieren einer auf dem Smartphone installierten Anwendung ("App"),** welche für die Verarbeitung der Sensormesswerte vorgesehen ist. In der Anwendung ist eine Untergrenze für die Wiederauffüllungszeit voreingestellt. Zum Beispiel könnte eine Untergrenze für die Wiederauffüllungszeit von 25 Sekunden voreingestellt sein. Falls erforderlich kann der Benutzer diese Voreinstellung entsprechend den spezifischen medizinischen Gegebenheiten individuell anpassen. Eine solche individuelle Anpassung könnte basierend auf dem Ergebnis (das heißt der Wiederauffüllungszeit) einer im Vorfeld der Kompressionstherapie vorgenommenen photoplethysmographischen Untersuchung erfolgen, welche auch mit der Sensoreinheit und dem Smartphone des vorliegenden Kompressionstherapiesystems durchgeführt werden kann. Wenn bei einem Patienten vor der Kompressionstherapie eine Wiederauffüllungszeit von zum Beispiel nur 10 Sekunden bestimmt wird, könnte die voreingestellte Untergrenze für die Wiederauffüllungszeit von 25 Sekunden auf 20 Sekunden herabgesetzt werden, da die Verlängerung der Wiederauffüllungszeit um 10 Sekunden im diesem Fall bereits einen zufriedenstellenden und gegebenenfalls maximalen therapeutischen Erfolg darstellen könnte. Zudem ist in der Anwendung eine gleichfalls anpassbare Obergrenze für die Wiederauffüllungszeit von beispielsweise 60 Sekunden voreingestellt. Die voreingestellte Obergrenze kann also ebenso wie die voreingestellte Untergrenze von dem Benutzer entsprechend den spezifischen medizinischen Gegebenheiten individuell angepasst werden.
   iv. **Starten einer Kontrollmessung** mit Hilfe der Anwendung. Der Patient führt dann ein Bewegungsprogramm mit einer anschließenden Ruhephase durch. Messdaten von dem photoplethysmographischen Sensor werden dabei von der Sensoreinheit an das Smartphone übertragen. Im Ergebnis wird dem Benutzer die Wiederauffüllungszeit angezeigt. Falls diese die eingestellte Untergrenze unterschreitet oder die eingestellte Obergrenze überschreitet, wird ein entsprechendes Alarmsignal ausgegeben. Damit wird sichergestellt, dass der Benutzer die unzureichende Wirksamkeit und/oder die mögliche Gefahrensituation ausgehend von der Kompressionstherapie in jedem Fall zur Kenntnis nimmt.
   v. **Falls ein Alarmsignal ausgegeben wird, kann eine entsprechende Maßnahme zur Beseitigung des Alarmzustandes erfolgen.** Zum Beispiel kann bei einer Unterschreitung der Untergrenze, also einer zu niedrigen gemessenen Wiederauffüllungszeit, die Kompressionsbinde abgenommen und mit höherem Zug wieder neu angelegt werden. Bei einer Überschreitung der Obergrenze, also einer zu hohen gemessenen Wiederauffüllungszeit, kann die Kompressionsbinde abgenommen und mit geringerem Zug wieder neu angelegt werden. Falls kein Alarmsignal ausgegeben wird, kann die Kompressionstherapie fortgesetzt werden. Der Benutzer hat dann Gewissheit, dass die Kompressionstherapie therapeutisch wirksam und sicher ist.
   vi. **Zu einem späteren, von dem Benutzer frei wählbaren Zeitpunkt kann eine erneute Kontrollmessung durchgeführt werden.** Diese Kontrollmessung muss wie die Kontrollmessung im obigen Schritt iv. vom Benutzer initiiert werden, denn die Datenübertragung und die Datenüberprüfung findet sinnvollerweise nur im Zusammenhang mit der zuvor genannten speziellen Bewegungs- und Ruhephase auf Abruf statt. Außerdem kann dadurch die Menge der übertragenen Messdaten verringert und der Energieverbrauch der Sensoreinheit minimiert werden. Vorteilhafterweise kann die Anwendung nach der erstmaligen Aktivierung auf dem Smartphone geöffnet bleiben, so dass sie den Benutzer erinnern kann, wenn eine erneute Kontrollmessung vorgenommen werden sollte (beispielsweise in Abständen von zwei Stunden).

Gegebenenfalls ist es auch möglich, zuerst den Unterschenkel mit der Kompressionsbinde zu umwickeln und danach die Sensoreinheit außen an der Binde zu befestigen. Voraussetzung hierfür ist, dass die Binde noch ausreichend infrarotes Licht für die Bestimmung der Wiederauffüllungszeit hindurchtreten lässt.

## Patentansprüche

1. System für eine Kompressionstherapie, umfassend
- ein Kompressionsmittel,
- einen photoplethysmographischen Sensor (3),
- einen Computer,
wobei Messwerte des photoplethysmographischen Sensors (3) an den Computer übertragen werden können, und
wobei der Computer Mittel zur Ausführung eines Verfahrens zur Überprüfung der Kompressionstherapie umfasst, wobei das Verfahren die folgenden Schritte umfasst:
i. Empfangen von Messwerten des photoplethysmographischen Sensors (3) während der Kompressionstherapie,
ii. Bestimmen einer Wiederauffüllungszeit anhand der empfangenen Messwerte des photoplethysmographischen Sensors (3),
iii. Prüfen, ob die bestimmte Wiederauffüllungszeit eine vorgegebene Untergrenze unterschreitet,
iv. optional Ausgabe eines Alarms, wenn die Prüfung ergibt, dass die vorgegebene Untergrenze unterschritten wird,
v. Prüfen, ob die bestimmte Wiederauffüllungszeit eine vorgegebene Obergrenze überschreitet,
vi. optional Ausgabe eines Alarms, wenn die Prüfung ergibt, dass die vorgegebene Obergrenze überschritten wird.

2. System nach Anspruch 1, wobei das Kompressionsmittel eine Kompressionsbinde, ein Kompressionsschlauch oder ein Kompressionsstrumpf ist.

3. System nach Anspruch 1 oder 2, wobei der photoplethysmographische Sensor (3) eine Lichtquelle für infrarotes Licht und einen Photodetektor umfasst, wobei die Lichtquelle vorzugsweise eine Leuchtdiode ist und der Photodetektor vorzugsweise eine Photodiode ist.

4. System nach einem der vorangehenden Ansprüche, wobei das System eine Sensoreinheit (1, 6) mit einem Substrat (2) umfasst, wobei der photoplethysmographische Sensor (3) auf dem Substrat (2) angeordnet ist.

5. System nach Anspruch 4, wobei es sich bei dem Substrat (2) um eine Leiterplatte, insbesondere um eine flexible Leiterplatte, handelt.

6. System nach Anspruch 4 oder 5, wobei die Sensoreinheit (1, 6) eine Datenerfassungseinheit (5) umfasst, wobei die Datenerfassungseinheit (5) dafür ausgebildet ist die Messwerte von dem photoplethysmographischen Sensor (3) zu erfassen und an den Computer zu übertragen.

7. System nach einem der Ansprüche 4 bis 6, wobei die Sensoreinheit (1, 6) adhäsiv ausgebildet ist und losgelöst von dem Kompressionsmittel bereitgestellt wird, wobei die Sensoreinheit (1, 6) vorzugsweise eine adhäsive Rückschicht (7) umfasst.

8. System nach einem der Ansprüche 4 bis 6, wobei die Sensoreinheit (1, 6) mit dem Kompressionsmittel herstellerseitig verbunden ist.

9. System nach einem der vorangehenden Ansprüche, wobei es sich bei dem Computer um ein mobiles Gerät, insbesondere ein Smartphone oder ein Tablet, handelt.

10. System nach einem der vorangehenden Ansprüche, wobei die Übertragung der Messwerte des photoplethysmographischen Sensors (3) an den Computer kabellos erfolgt, wobei die Übertragung vorzugsweise über eine Nahfeldkommunikation, Bluetooth oder ein drahtloses lokales Netzwerk erfolgt.

11. System nach einem der vorangehenden Ansprüche, wobei das Empfangen der Messwerte des photoplethysmographischen Sensors (3) unmittelbar nachdem die Messwerte von dem Sensor (3) erfasst wurden erfolgt.

12. System nach einem der vorangehenden Ansprüche, wobei das Empfangen der Messwerte des photoplethysmographischen Sensors (3) auf Befehl eines Benutzers hin erfolgt.

13. System nach einem der vorangehenden Ansprüche, wobei die Untergrenze aus einem Bereich von 10 Sekunden bis 25 Sekunden, bevorzugt von 15 Sekunden bis 25 Sekunden, mehr bevorzugt von 20 Sekunden bis 25 Sekunden und besonders bevorzugt von 23 Sekunden bis 25 Sekunden ausgewählt ist, wobei die Untergrenze insbesondere 25 Sekunden beträgt.

14. System nach einem der vorangehenden Ansprüche, wobei die Obergrenze aus einem Bereich von 30 Sekunden bis 180 Sekunden, bevorzugt von 35 Sekunden bis 90 Sekunden, mehr bevorzugt von 40 Sekunden bis 90 Sekunden und besonders bevorzugt von 45 Sekunden bis 90 Sekunden ausgewählt ist, wobei die Obergrenze insbesondere 60 Sekunden beträgt.

15. Computerprogrammprodukt, umfassend Befehle, die bei der Ausführung des Programmes durch ein System nach einem der Ansprüche 1 bis 14 dieses veranlassen, die nachfolgenden Schritte i. bis vii., die nachfolgenden Schritte iii. bis vii. oder zumindest die nachfolgenden Schritte iii., iv. und vi. eines computerimplementierten Verfahrens zur Überprüfung einer Kompressionstherapie auszuführen:
i. Erzeugen von Messwerten mit einem photoplethysmographischen Sensor (3) während der Kompressionstherapie, um die Wirksamkeit der Kompressionstherapie zu überprüfen,
ii. Übertragen der Messwerte des photoplethysmographischen Sensors (3) während der Kompressionstherapie an einen Computer,
iii. Bestimmen einer Wiederauffüllungszeit anhand der Messwerte des photoplethysmographischen Sensors (3) durch den Computer,
iv. Prüfen durch den Computer, ob die bestimmte Wiederauffüllungszeit eine vorgegebene Untergrenze unterschreitet,
v. optional Ausgabe eines Alarms durch den Computer, wenn die Prüfung ergibt, dass die vorgegebene Untergrenze unterschritten wird,
vi. Prüfen durch den Computer, ob die bestimmte Wiederauffüllungszeit eine vorgegebene Obergrenze überschreitet,
vii. optional Ausgabe eines Alarms durch den Computer, wenn die Prüfung ergibt, dass die vorgegebene Obergrenze überschritten wird.

16. Computerlesbarer Datenträger, auf dem das Computerprogrammprodukt nach Anspruch 15 gespeichert ist.

17. Datenträgersignal, das das Computerprogrammprodukt nach Anspruch 15 überträgt.

## Claims

1. System for a compression therapy, comprising
- a compression means,
- a photoplethysmographic sensor (3),
- a computer,
wherein measured values of the photoplethysmographic sensor (3) can be transmitted to the computer, and wherein the computer comprises means for performing a method for checking the compression therapy, wherein the method comprises the following steps:
i. receiving measured values of the photoplethysmographic sensor (3) during the compression therapy,
ii. determining a refilling time on the basis of the received measured values of the photoplethysmographic sensor (3),
iii. checking whether the determined refilling time undershoots a predefined lower limit,
iv. optionally outputting an alarm if the check reveals that the predefined lower limit is undershot,
v. checking whether the determined refilling time exceeds a predefined upper limit,
vi. optionally outputting an alarm if the check reveals that the predefined upper limit is exceeded.

2. System according to Claim 1, wherein the compression means is a compression bandage, a compression sleeve or a compression stocking.

3. System according to Claim 1 or 2, wherein the photoplethysmographic sensor (3) comprises a light source for infrared light and a photodetector, wherein the light source is preferably a light-emitting diode and the photodetector is preferably a photodiode.

4. System according to any of the preceding claims, wherein the system comprises a sensor unit (1, 6) having a substrate (2), wherein the photoplethysmographic sensor (3) is arranged on the substrate (2).

5. System according to Claim 4, wherein the substrate (2) is a printed circuit board, in particular a flexible printed circuit board.

6. System according to Claim 4 or 5, wherein the sensor unit (1, 6) comprises a data acquisition unit (5), wherein the data acquisition unit (5) is designed to acquire the measured values from the photoplethysmographic sensor (3) and to transmit them to the computer.

7. System according to any of Claims 4 to 6, wherein the sensor unit (1, 6) is of adhesive design and is provided in a manner detached from the compression means, wherein the sensor unit (1, 6) preferably comprises an adhesive back layer (7).

8. System according to any of Claims 4 to 6, wherein the sensor unit (1, 6) is connected to the compression means by the manufacturer.

9. System according to any of the preceding claims, wherein the computer is a mobile device, in particular a smartphone or a tablet.

10. System according to any of the preceding claims, wherein the transmission of the measured values of the photoplethysmographic sensor (3) to the computer is effected wirelessly, wherein the transmission is preferably effected by way of near field communication, Bluetooth or a wireless local network.

11. System according to any of the preceding claims, wherein receiving the measured values of the photoplethysmographic sensor (3) takes place directly after the measured values have been acquired by the sensor (3).

12. System according to any of the preceding claims, wherein receiving the measured values of the photoplethysmographic sensor (3) takes place at the command of a user.

13. System according to any of the preceding claims, wherein the lower limit is selected from a range of 10 seconds to 25 seconds, preferably of 15 seconds to 25 seconds, more preferably of 20 seconds to 25 seconds, and particularly preferably of 23 seconds to 25 seconds, wherein the lower limit is in particular 25 seconds.

14. System according to any of the preceding claims, wherein the upper limit is selected from a range of 30 seconds to 180 seconds, preferably of 35 seconds to 90 seconds, more preferably of 40 seconds to 90 seconds, and particularly preferably of 45 seconds to 90 seconds, wherein the upper limit is in particular 60 seconds.

15. Computer program product, comprising instructions which, when the program is executed by a system according to any of Claims 1 to 14, cause said system to perform the following steps i. to vii., the following steps iii. to vii. or at least the following steps iii., iv. and vi. of a computer-implemented method for checking a compression therapy:
i. generating measured values by way of a photoplethysmographic sensor (3) during the compression therapy in order to check the effectiveness of the compression therapy,
ii. transmitting the measured values of the photoplethysmographic sensor (3) during the compression therapy to a computer,
iii. determining a refilling time on the basis of the measured values of the photoplethysmographic sensor (3) by means of the computer,
iv. checking, by means of the computer, whether the determined refilling time undershoots a predefined lower limit,
v. optionally outputting an alarm by means of the computer if the check reveals that the predefined lower limit is undershot,
vi. checking, by means of the computer, whether the determined refilling time exceeds a predefined upper limit,
vii. optionally outputting an alarm by means of the computer if the check reveals that the predefined upper limit is exceeded.

16. Computer-readable data carrier on which the computer program product according to Claim 15 is stored.

17. Data carrier signal which transmits the computer program product according to Claim 15.

## Revendications

1. Système pour une thérapie de compression, comprenant
- un moyen de compression,
- un capteur photopléthysmographique (3),
- un ordinateur,
dans lequel les valeurs de mesure du capteur photopléthysmographique (3) peuvent être transmises à l'ordinateur, et
dans lequel l'ordinateur comprend des moyens pour exécuter un procédé permettant de contrôler la thérapie de compression, le procédé comprenant les étapes suivantes consistant à :
i. recevoir des valeurs de mesure du capteur photopléthysmographique (3) pendant la thérapie de compression,
ii. déterminer un délai de reconstitution à l'aide des valeurs de mesure reçues du capteur photopléthysmographique (3),
iii. contrôler si le délai de reconstitution déterminé soupasse une limite inférieure prédéfinie,
iv. en option, sortir une alarme s'il résulte du contrôle que la limite inférieure prédéfinie est soupassée,
v. contrôler si le délai de reconstitution déterminé dépasse une limite supérieure prédéfinie,
vi. en option, sortir une alarme s'il résulte du contrôle que la limite supérieure prédéfinie est dépassée.

2. Système selon la revendication 1, dans lequel le moyen de compression est une bande de compression, un tube de compression ou un bas de compression.

3. Système selon la revendication 1 ou 2, dans lequel le capteur photopléthysmographique (3) comprend une source de lumière pour une lumière infrarouge et un photodétecteur, la source de lumière étant de préférence une diode électroluminescente et le photodétecteur étant de préférence une photodiode.

4. Système selon l'une quelconque des revendications précédentes, dans lequel le système comprend une unité de capteur (1, 6) pourvue d'un substrat (2), le capteur photopléthysmographique (3) étant disposé sur le substrat (2).

5. Système selon la revendication 4, dans lequel le substrat (2) est une carte de circuits imprimés, en particulier une carte de circuits imprimés flexible.

6. Système selon la revendication 4 ou 5, dans lequel l'unité de capteur (1, 6) comprend une unité de détection de données (5), dans lequel l'unité de détection de données (5) est réalisée pour détecter les valeurs de mesure du capteur photopléthysmographique (3) et pour les transmettre à l'ordinateur.

7. Système selon l'une quelconque des revendications 4 à 6, dans lequel l'unité de capteur (1, 6) est réalisée de manière adhésive et est fournie de manière détachée du moyen de compression, dans lequel l'unité de capteur (1, 6) comprend de préférence une couche arrière adhésive (7).

8. Système selon l'une quelconque des revendications 4 à 6, dans lequel l'unité de capteur (1, 6) est reliée côté fabricant au moyen de compression.

9. Système selon l'une quelconque des revendications précédentes, dans lequel l'ordinateur est un appareil mobile, en particulier un smartphone ou une tablette.

10. Système selon l'une quelconque des revendications précédentes, dans lequel la transmission des valeurs de mesure du capteur photopléthysmographique (3) à l'ordinateur est effectuée sans fil, la transmission étant effectuée de préférence par une communication en champ proche, par Bluetooth ou par un réseau local sans fil.

11. Système selon l'une quelconque des revendications précédentes, dans lequel la réception des valeurs de mesure du capteur photopléthysmographique (3) a lieu immédiatement après que les valeurs de mesure ont été détectées par le capteur (3).

12. Système selon l'une quelconque des revendications précédentes, dans lequel la réception des valeurs de mesure du capteur photopléthysmographique (3) a lieu suite à une instruction d'un utilisateur.

13. Système selon l'une quelconque des revendications précédentes, dans lequel la limite inférieure est sélectionnée dans une plage de 10 secondes à 25 secondes, de préférence de 15 secondes à 25 secondes, de plus grande préférence de 20 secondes à 25 secondes, et de manière particulièrement préférée de 23 secondes à 25 secondes, la limite inférieure étant en particulier égale à 25 secondes.

14. Système selon l'une quelconque des revendications précédentes, dans lequel la limite supérieure est sélectionnée dans une plage de 30 secondes à 180 secondes, de préférence de 35 secondes à 90 secondes, de plus grande préférence de 40 secondes à 90 secondes, et de manière particulièrement préférée de 45 secondes à 90 secondes, la limite supérieure étant en particulier égale à 60 secondes.

15. Produit de programme informatique, comprenant des instructions qui, lorsque le programme est exécuté par un système selon l'une quelconque des revendications 1 à 14, font que celui-ci exécute les étapes i. à vii. suivantes, les étapes iii. à vii. suivantes ou au moins les étapes suivantes iii., iv. et vi. d'un procédé mis en œuvre sur ordinateur pour contrôler une thérapie de compression :
i. générer des valeurs de mesure à l'aide d'un capteur photopléthysmographique (3) pendant la thérapie de compression afin de contrôler l'efficacité de la thérapie de compression,
ii. transmettre les valeurs de mesure du capteur photopléthysmographique (3) à un ordinateur pendant la thérapie de compression,
iii. déterminer à l'aide de l'ordinateur un délai de reconstitution au moyen des valeurs de mesure du capteur photopléthysmographique (3),
iv. contrôler à l'aide de l'ordinateur si le délai de reconstitution déterminé soupasse une limite inférieure prédéfinie,
v. en option, sortir une alarme à l'aide de l'ordinateur s'il résulte du contrôle que la limite inférieure prédéfinie est soupassée,
vi. contrôler à l'aide de l'ordinateur si le délai de reconstitution déterminé dépasse une limite supérieure prédéfinie,
vii. en option, sortir une alarme à l'aide de l'ordinateur s'il résulte du contrôle que la limite supérieure prédéfinie est dépassée.

16. Support de données lisible par ordinateur sur lequel est stocké le produit de programme informatique selon la revendication 15.

17. Signal porteur de données qui transmet le produit de programme informatique selon la revendication 15.
